(19) Europäisches Patentamt
European Patent Office
Office européen des brevets

(11) EP 0 839 146 B1

(12) EUROPEAN PATENT SPECIFICATION

(45) Date of publication and mention of the grant of the patent:
**13.09.2000 Bulletin 2000/37**

(21) Application number: **96924983.8**

(22) Date of filing: **19.07.1996**

(51) Int Cl.[7]: **C07D 405/14**, A61K 31/495

(86) International application number:
**PCT/GB96/01714**

(87) International publication number:
**WO 97/03982 (06.02.1997 Gazette 1997/07)**

(54) **PIPERAZINE DERIVATIVES AND THEIR USE AS 5-HT1A ANTAGONISTS**
PIPERAZIN-DERIVATE UND IHRE VERWENDUNG ALS 5-HT1A-ANTAGONISTE
DERIVES DE PIPERAZINE ET LEUR UTILISATION EN TANT QU'ANTAGONISTES 5-HT1A

(84) Designated Contracting States:
**AT BE CH DE DK ES FI FR GB GR IE IT LI LU NL PT SE**
Designated Extension States:
**AL LT LV SI**

(30) Priority: **20.07.1995 GB 9514901**

(43) Date of publication of application:
**06.05.1998 Bulletin 1998/19**

(73) Proprietor: **AMERICAN HOME PRODUCTS CORPORATION**
**Madison, New Jersey 07940-0874 (US)**

(72) Inventors:
- **FENSOME, Andrew**
  **Wayne, PA 19087 (US)**
- **KELLY, Michael, Gerard**
  **Plainsboro, NJ 05836 (US)**
- **MANSELL, Howard, Langham**
  **Burnham, Buckinghamshire SL1 7AW (GB)**

(74) Representative:
**Walters, Philip Bernard William et al**
**Wyeth Laboratories,**
**Patents & Trade Marks Department,**
**Huntercombe Lane South,**
**Taplow**
**Maidenhead, Berkshire SL6 0PH (GB)**

(56) References cited:
**EP-A- 0 512 755**
**WO-A-95/33725**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 839 146 B1

## Description

This invention relates to novel piperazine derivatives, to processes for their preparation, to their use and to pharmaceutical compositions containing them. The novel compounds are useful as 5-$HT_{1A}$ binding agents, particularly as 5-$HT_{1A}$-antagonists.

EP-A-0512755 discloses compounds of the general formula

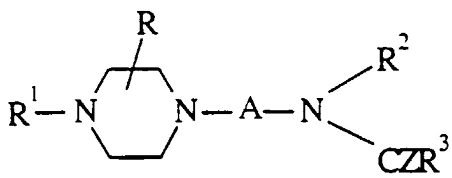

(I)

and the pharmaceutically acceptable acid addition salts thereof wherein

A is an alkylene chain of 2 to 4 carbon atoms optionally substituted by one or more lower alkyl groups,

Z is oxygen or sulphur,

R is hydrogen or lower alkyl,

$R^1$ is a mono or bicyclic aryl or heteroaryl radical,

$R^2$ is a mono or bicyclic heteroaryl radical

and
$R^3$ is hydrogen, lower alkyl, cycloalkyl, cycloalkenyl, cycloalkyl(lower)alkyl, aryl, aryl(lower)alkyl, heteroaryl, heteroaryl (lower)alkyl, a group of formula $-NR^4R^5$ [where $R^4$ is hydrogen, lower alkyl, aryl or aryl(lower)alkyl and $R^5$ is hydrogen, lower alkyl, -CO(lower)alkyl, aryl, COaryl, aryl(lower)alkyl, cycloalkyl, or cycloalkyl-(lower)alkyl or $R^4$ and $R^5$ together with the nitrogen atom to which they are both attached represent a saturated heterocyclic ring which may contain a further hetero atom] or a group of formula $OR^6$ [where $R^6$ is lower alkyl, cycloalkyl, cycloalkyl(lower)alkyl, aryl, aryl (lower)alkyl, heteroaryl or heteroaryl(lower)alkyl].

The compounds are disclosed as 5-$HT_{1A}$ binding agents, particularly 5-$HT_{1A}$ antagonists, e.g. for the treatment of CNS disorders, for example anxiety.

We have now found that a compound falling within formula (I), but not specifically disclosed in EP-A-0512755, and its pharmaceutically acceptable salts have specially advantageous properties that make the compounds particularly useful as 5-$HT_{1A}$ antagonists in the treatment of CNS disorders when administered by the oral route.

The novel compounds of the invention are the compound of the general formula

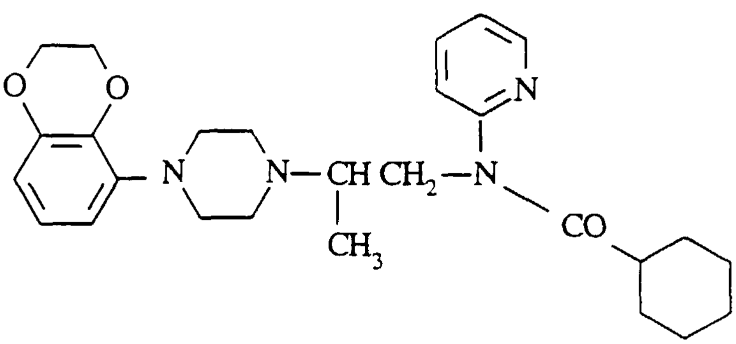

(A)

and the pharmaceutically acceptable acid addition salts thereof.

The compound of formula (A) contains an asymmetric carbon atom so that it may exist in different stereoisomeric forms e.g. as a racemate or as optically active forms. The preferred compounds are:

R-N-[1-[2-[1-[4-[50-(2,3-dihydro[1,4]benzodioxinyl)]piperazinyl]propyl]-N-(2-pyridyl)-cyclohexanecarboxamide (hereinafter referred to as compound A1) and the pharmacologically acceptable acid addition salts thereof.

We have found that the novel compounds of the present invention are potent 5-$HT_{1A}$ binding agents, being similar to potency to the most potent of the compounds disclosed in EP-A-0512755. The novel compounds selectively bind to the 5-$HT_{1A}$ receptors to a much greater extent than they bind to other receptors, such as $\alpha_1$ receptors. The novel compounds are 5-$HT_{1A}$ antagonists when tested by standard pharmacological procedures. Surprisingly we have found that the novel compounds are particularly potent as 5-$HT_{1A}$ antagonists when administered by the oral route. The novel compounds are many more times more potent, as 5-$HT_{1A}$ antagonists, when administered by the oral route, than the most potent compounds of EP-A-512755. The 5-$HT_{1A}$ antagonists of the present invention can be used for the treatment of CNS disorders, such as schizophrenia (and other psychotic disorders such as paranoia and mano-depressive illness) and anxiety (e.g. generalised anxiety disorders, panic attacks and obsessive compulsive disorders), in mammals, particularly humans. The 5-$HT_{1A}$-antagonists can also be used as antidepressants, hypotensives and as agents for regulating the sleep/wake cycle, feeding behaviour and/or sexual function and as cognition enhancing agents. The increased oral bioavailability of the novel compounds of the invention, compared to that of the class of compounds disclosed in EP-A-512755 is particularly advantageous in that a much smaller dose of compound can be administered orally to produce a similar therapeutic effect.

Tables I and II below summarise the 5-$HT_{1A}$ receptor binding activity, the al receptor binding activity, the binding selectivity (i.e. the ratio of 5-$HT_{1A}$ binding to $\alpha_1$-binding) and the 5-$HT_{1A}$ antagonist activity by the oral route of the compounds disclosed in EP-A-512755 and the novel compounds of the present invention.

TABLE I

| 1 | 2 | 3 | 4 | 5 | 6 |
|---|---|---|---|---|---|
| **Compounds of prior art (Example No of EP-0512755** | **5$HT_{1A}$ binding $IC_{50}$ (nM)** | **$\alpha_1$ binding $IC_{50}$ (nM)** | **Ratio 5$HT_{1A}$/$\alpha_1$** | **5-$HT_{1A}$-antagonist activity MED mg/kg po** | **5-$HT_{1A}$-antagonist Ratio*** |
| 3 | 2.2 | 230 | 105 | 1 | 4.2 |
| 5 | 12 | 230 | 19.2 | >10 | |
| 6 | 60 | 245 | 4 | | |
| 8 | 90 | 140 | 2.5 | | |
| 11 | 1 | 197 | 197 | 10 | |
| 17 | 3.1 | 63 | 19.3 | 1 | 7.6 |
| 20 | 4.1 | 385 | 93.9 | >10 | |
| 30 | 14 | 74 | 5.3 | 10 | |
| 33 | 1.4 | 125 | 89.3 | 10 | 7.0 |
| 46 | 2.3 | 798 | 346.9 | 3 | |
| 47 | 4.9 | 64 | 13 | 3-10 | |
| 48 | 6.4 | 126 | 19.7 | | |
| 49 | 2.7 | 1403 | 519.6 | 10 | |
| 50 | 4 | 40 | 10 | | |
| 51 | 3.7 | 46 | 12.4 | <10 | |
| 52 | 147 | | | | |
| 53 | 8 | 558 | 69.8 | 10 | |
| 54 | 175 | | | | |
| 55 | 2.3 | 688 | 299.1 | 1 | 3.2 |
| 56 | 2.7 | 56 | 20.7 | | |

TABLE I (continued)

| 1 | 2 | 3 | 4 | 5 | 6 |
|---|---|---|---|---|---|
| **Compounds of prior art (Example No of EP-0512755** | **$5HT_{1A}$ binding $IC_{50}$ (nM)** | **$\alpha_1$ binding $IC_{50}$ (nM)** | **Ratio $5HT_{1A}/\alpha_1$** | **$5\text{-}HT_{1A}$-antagonist activity MED mg/kg po** | **$5\text{-}HT_{1A}$-antagonist Ratio*** |
| 57 | 12.7 | 281 | 22.1 | | |
| 58 | 16 | 28 | 1.75 | 1-3 | |
| 59 | 67 | | | | |
| 60 | 3 | 312 | 104 | 0.3 | 7.4 |
| 61 | 18 | 136 | 7.1 | | |
| 62 | 10 | 144 | 14.4 | | |
| 63 | 131 | | | | |
| 64 | 35 | | | | |
| 65 | 13 | 115 | 8.8 | | |
| 66 | 1.8 | 28 | 15.5 | | |

Table II

| 1 | 2 | 3 | 4 | 5 | 6 |
|---|---|---|---|---|---|
| **Compounds of the invention** | **$5HT_{1A}$ binding $IC_{50}$ (nM)** | **$\alpha_1$ binding $IC_{50}$ (nM)** | **Ratio $5HT_{1A}/\alpha_1$** | **$5\text{-}HT_{1A}$-antagonist activity MED mg/kg po** | **$5\text{-}HT_{1A}$-antagonist Ratio*** |
| A1 | 4.3 | 625 | 145 | 1 | 20 |

***($ED_{50}$ antagonist @ 3mg/kg po)/$ED_{50}$ vehicle**

The compounds were tested for $5\text{-}HT_{1A}$ binding affinity (column 2) in rat hippocampal membrane homogenate by the method of B.S. Alexander and M.D. Wood, J. Pharm. Pharmacol., 1988, 40, 888-891.

The compounds were tested for $\alpha_1$ binding affinity (column 3) by the procedure of A.L. Marrow et al, Mol. Pharmacol., 1986, 29, 321.

$5\text{-}HT_{1A}$ receptor antagonist activity (columns 5 and 6) is assessed by the ability of the selective $5\text{-}HT_{1A}$ receptor agonist, 8-OH-DPAT, to induce 'the 8-OH-DPAT syndrome' in rats characterised by extended flat body posture, forepaw treading and hyperlocomotion. The 8-OH-DPAT syndrome is scored as present (definite syndrome response) or absent (equivocal or no syndrome response) during the period 0-5 min following intravenous (i.v.) administration of the test agonist via a lateral tail vein.

A range of doses, on a logarithmic scale and encompassing the expected $ED_{50}$, were chosen for the test agonist following preliminary evaluation. The first animal received a dose of the test agonist approximating the expected ED50. If the animal responded (syndrome present) the subsequent animal received the next lowest dose on the scale, whereas if the animal did not respond (syndrome absent or equivocal) the next animal received the next highest dose of the chosen scale. This procedure was repeated for a minimum of 10 animals, with animals being tested sequentially.

Test antagonists were administered orally (p.o.) 60 min, before i.v. administration of 8-OH-DPAT. $ED_{50}$ values for 8-OH-DPAT were determined for the different pretreatment groups using the sequential up/down procedure as described above.

Minimum effective doses (MEDs) are taken as the lowest dose tested at which confidence limits for the $ED_{50}$ values for the antagonist and vehicle pretreated groups do not overlap.

The response to the selective $5\text{-}HT_{1A}$ receptor agonist, 8-OH-DPAT, is represented as an $ED_{50}$ value for induction of the 8-OH-DPAT syndrome, which is determined using the sequential up/down analysis following intravenous administration. $5\text{-}HT_{1A}$ antagonist activity is determined by the ability of the test compound to antagonise the response to 8-OH-DPAT, i.e. to increase the $ED_{50}$ for induction of the 8-OH-DPAT syndrome compared with vehicle

pretreatment. The ratios represent the $ED_{50}$ value for 8-OH-DPAT following pretreatment with the test compound at 3 mg/kg p.o. ($ED_{50}$ antagonist) divided by the $ED_{50}$ value for 8-OH-DPAT following pretreatment with vehicle ($ED_{50}$ vehicle).

i.e. ratio = ($ED_{50}$ antagonist @ 3 mg/kg p.o.) / ($ED_{50}$ vehicle)

By calculating the ratio for all compounds at the same dose, i.e. 3 mg/kg p.o., a direct comparison can be made as to their effect at that dose. Thus the larger the ratio, the greater the difference between the ED50 values following 5-$HT_{1A}$ antagonist and vehicle pretreatment, hence the greater the antagonist effect. In summary the greater the ratio the more potent the 5-$HT_{1A}$ antagonist is following oral administration.

Those compounds of EP-A-512755 which showed the best 5-$HT_{1A}$ affinity and 5-$HT_{1A}/\alpha_1$ selectivity were tested for 5-$HT_{1A}$ antagonist activity (column 5) and those compounds which showed the best 5-$HT_{1A}$ antagonist activity (column 5) were further tested in the procedure of column 6. The results clearly show that the compound (A1) of the present invention has good 5-$HT_{1A}$ binding affinity and 5-$HT_{1A}/\alpha_1$ selectivity and shows a surprising increase in oral activity as a 5-$HT_{1A}$ antagonist compared to the class of compounds disclosed generally in EP-A-512755. As an illustration the oral 5-$HT_{1A}$ antagonist ratio for compound A1 (ratio 20) should be compared with that of the compound closest in structure disclosed in the prior art i.e. the compound of Example 17 having a ratio of 7.6.

Not only is the compound A1 surprisingly more potent orally than the compound of Example 17 it has a much better selectivity i.e. ratio of 145 compared to that of 19.3 for the prior art compound.

The compounds of the invention can be prepared by known methods from known starting materials or starting materials that may be prepared by conventional methods. For example the compounds may be prepared by the general methods disclosed in EP-A-0512755.

One method of preparing the compound of the invention comprises acylating an amine of formula

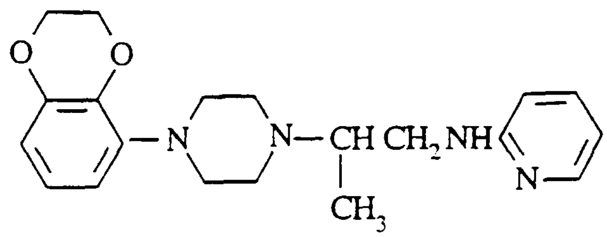

(B)

with cyclohexanecarboxylic acid or an acylating derivative thereof. Examples of acylating derivatives include the acid halides (e.g. acid chlorides), azides, anhydrides, imidazolides (e.g. obtained from carbonyldiimidazole), activated esters or O-acyl ureas obtained from a carbodiimide such as a dialkylcarbodiimide particularly cyclohexylcarbodiimide.

The starting amide of general formula (B) is a novel compound and is also provided by the present invention. It may be prepared by the general route disclosed in EP-A-0512755 e.g. the route exemplified below:-

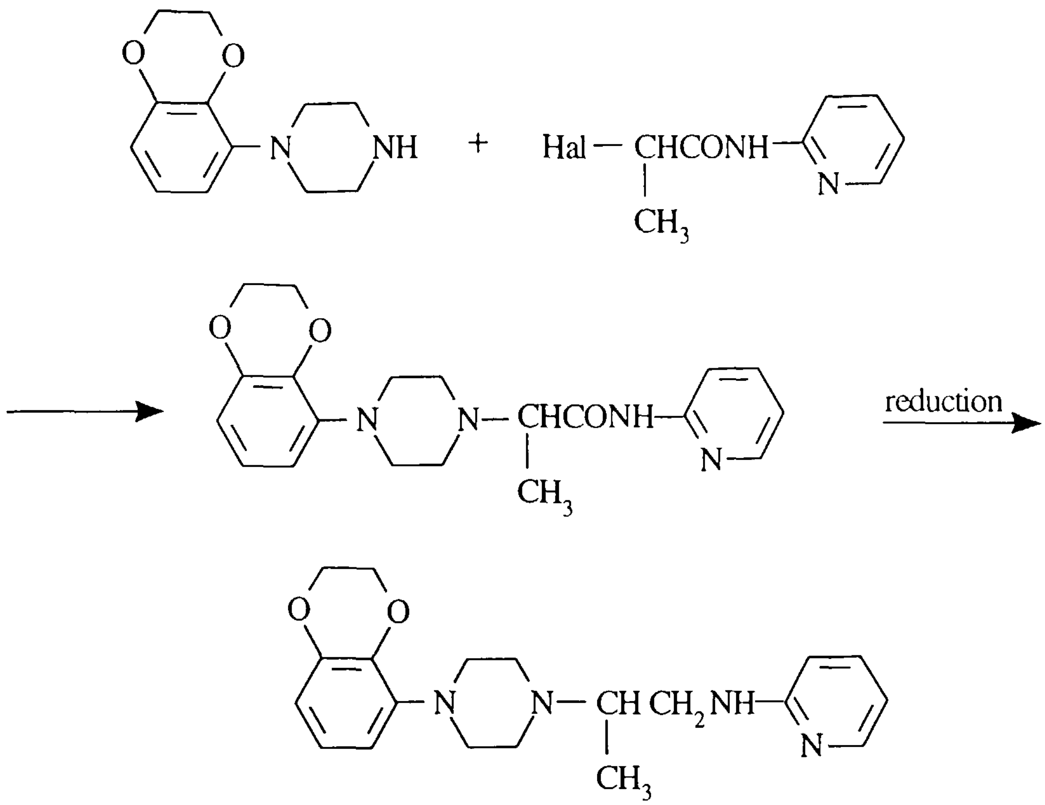


(C)

(where Hal is halo, particularly chloro or bromo). The reduction may be carried out with, for example a complex metal hydride, e.g. lithium aluminium hydride.

In an alternate method of preparing the starting materials of formula B the oxathiazolidine-2,2-dioxide of formula

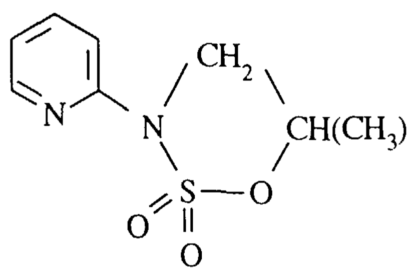

(D)

is reacted with 1-(2,3-dihydro-1,4-benzodioxin-5-yl)piperazine. The reaction and a process for the preparation of the sulphoxide are illustrated in the scheme below:

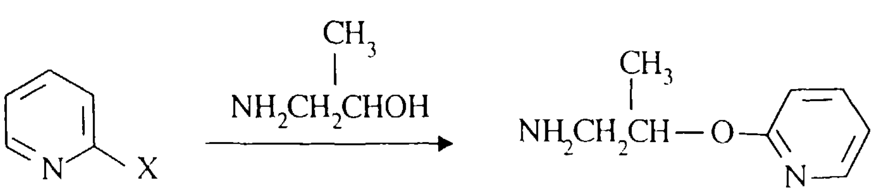

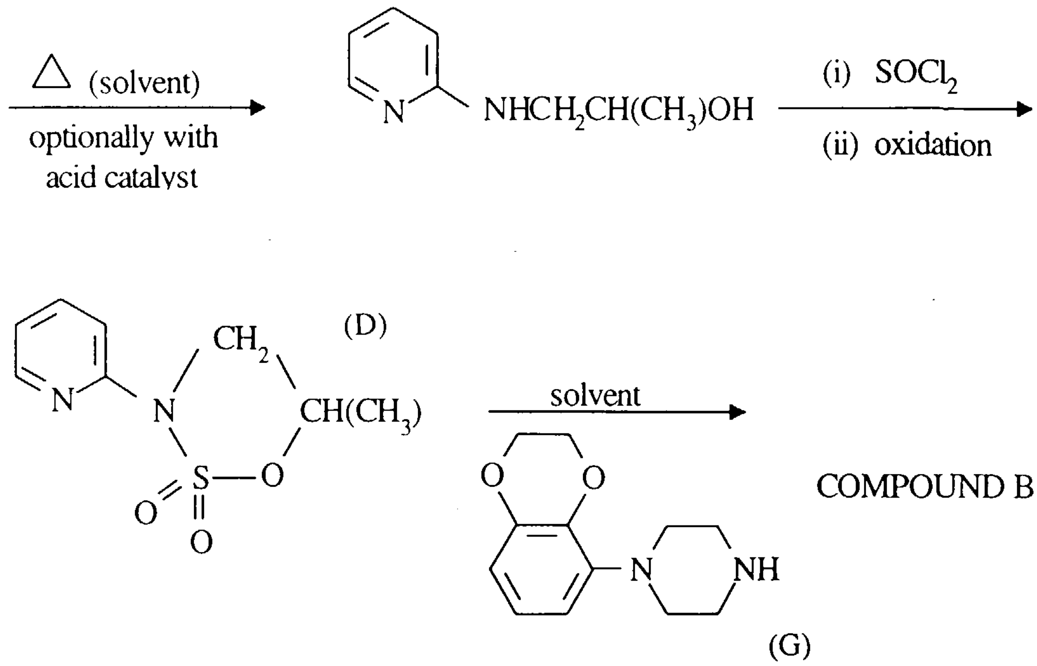


(where X is a leaving group, preferably chloro, bormo or fluoro).

Certain process steps in the above scheme and certain novel intermediates of the scheme are claimed in published PCT application WO 95/33725 (PCT/GB95/01256) in the name of John Wyeth & Brother Limited entitled "Novel Processes and Intermediates for the Preparation of Piperazine Derivatives". This application claims priority from British patent application No. 9411108.5 filed on 3 June 1994.

A further method of preparing the compounds of the invention comprises alkylating the amide of formula

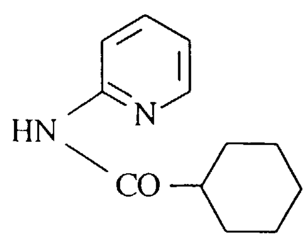
(E)

with an alkylating agent providing the group

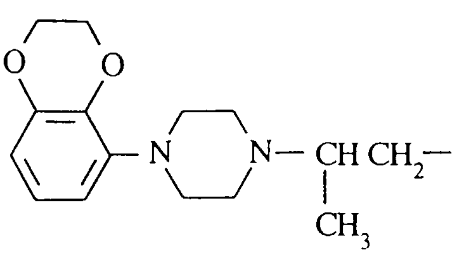
(F)

The alkylating agent may be, for example, a compound of formula

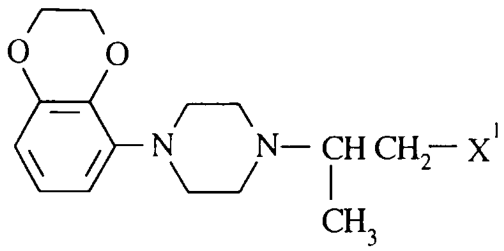

where $X^1$ is a leaving group such as halogen or an alkyl- or aryl- sulphonyloxy group.

A further method of preparing the compounds of the invention comprises alkylating a compound of formula

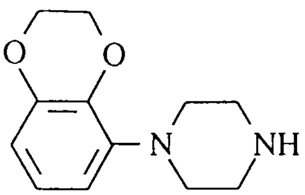

(G)

with a compound of formula

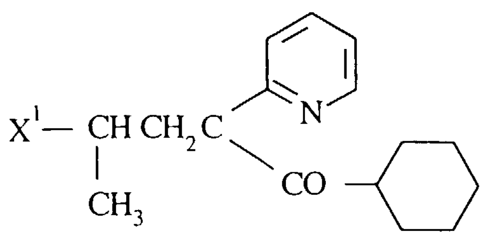

(H)

(where $X^1$ is as defined above).

The processes described above may be carried out to give a compound of the invention in the form of a free base or as an acid addition salt. If the compound of the invention is obtained as an acid addition salt, the free base can be obtained by basifying a solution of the acid addition salt. Conversely, if the product of the process is a free base an acid addition salt, particularly a pharmaceutically acceptable acid addition salt, may be obtained by dissolving the free base in a suitable organic solvent and treating the solution with an acid, in accordance with conventional procedures for preparing acid addition salts from base compounds.

Examples of acid addition salts are those formed from inorganic and organic acids, such as sulphuric, hydrochloric, hydrobromic, phosphoric, tartaric, fumaric, maleic, citric, acetic, formic, methanesulphonic, p-toluenesulphonic, oxalic and succinic acids.

The optically active form of the compound of the invention can be obtained by resolution of the racemates or by asymmetric synthesis or using readily available chiral precursors.

The invention also provides a pharmaceutical composition comprising a compound or a pharmaceutically acceptable acid addition salt thereof in association with a pharmaceutically acceptable carrier. Any suitable carrier known in the art can be used to prepare the pharmaceutical composition. In such a composition, the carrier is generally a solid or liquid or a mixture of a solid or liquid.

Solid form compositions include powders, granules, tablets, capsules (e.g. hard and soft gelatine capsules), suppositories and pessaries. A solid carrier can be, for example, one or more substances which may also act as flavouring agents, lubricants, solubilisers, suspending agents, fillers, glidants, compression aides, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above, e.g. cellulose derivatives, preferably sodium carboxymethyl cellulose solution), alcohols, (e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged composition, for example packeted powders, vials, ampoules, prefilled syringes or sachets containing liquid. The unit dosage form can be, for example, a capsule or tablet itself, or it can be the appropriate number of any such compositions in package form. The quantity of the active ingredient in unit dose of composition may be varied or adjusted from 0.5 mg or less to 750 mg or more, according to the particular need and the activity of the active ingredient.

The invention also provides the use of a compound of the present invention in the manufacture of a medicament for the treatment of CNS disorders in mammals.

The following Example illustrates the invention.

Example 1

(R)-N-[1-[2-[1-[4-[5-(2,3-dihydro[1,4]benzodioxinyl)]]piperazinyl]propyl]-N-(2-pyridyl)cyclohexanecarboxamide

Triethylamine (0.85 mL, 0.6g, 6mmol) and cyclohexanecarbonyl chloride (0.74 mL, 0.81g, 5.5mmol) were added dropwise with ice/water cooling to a stirred solution of (R)-2-[1-[4-[5-(2,3-dihydro[1,4]benzodioxinyl)]]piperazinyl]-N-(2-pyridyl)propylamine (1.77g, 5.0mmol) in dichloromethane (10 mL). [The (R)-2-[1-[4-[5-(2,3-dihydro[1,4]benzodioxinyl)]]piperazinyl]-N-(2-pyridyl)propylamine was prepared by reacting (S)-5-methyl-3-pyrid-2-yl[1,2,3]oxathiazolidine-2,2-oxide with 1-(2,3-dihydro-1,4-benzodioxan-5-yl)piperazine]. The suspension was stirred at 0°C for 1 h, and dichloromethane (25 mL) was added. The solution was washed with water (25 mL), dried ($Na_2SO_4$) and concentrated in vacuo to give an orange foam. The crude product was chromatographed on silica with eluant ethyl acetate : hexane (2 : 3 → 1 : 0) to give (R)-N-[1-[2-[1-[4-[5-(2,3-dihydro[1,4]benzodioxinyl)]]piperazinyl]]propyl]-N-(2-pyridyl)cyclohexanecarboxamide as the free base and as a white foam (2.09g). This foam was dissolved in methanol (100 mL), and the solution was acidified with ethereal hydrogen chloride and concentrated in vacuo. Acetonitrile was. added, the solution was concentrated, and the residue was triturated with ethyl acetate to give the title compound as the dihydrochloride hydrate (2.25g) m.p. 120-220°C

(Found: C, 58.7: H. 7.5; N, 9.8 $C_{27}H_{36}N_4O_3$,2HCl.$H_2O$ requires C, 58.4: H, 7.3; N, 10.1%); $[\alpha]_D^{28}$ + 26° (c 1.0 in MeOH).

## Claims

**1.** A compound of the general formula (A):

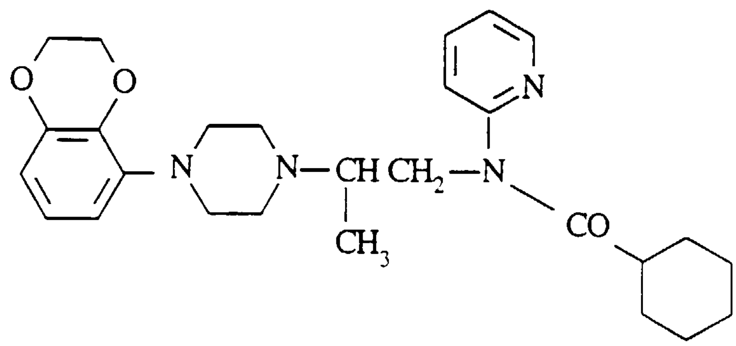

(A)

or a pharmaceutically acceptable acid addition salt thereof.

2. R-N-[1-[2-[1-[4-[5-(2,3-dihydro[1,4]benzodioxinyl)]piperazinyl]propyl]-N-(2-pyridyl)-cyclohexanecarboxamide or a pharmaceutically acceptable acid addition salt thereof.

3. A compound as claimed in claim 1 or claim 2 wherein the acid addition salt is a salt derived from sulphuric, hydrochloric, hydrobromic, phosphoric, tartaric, fumaric, maleic, citric, acetic, formic, methanesulphonic, p-toluenesulphonic. oxalic or succinic acid.

4. A pharmaceutical composition comprising a compound as claimed in any one of claims 1 to 3 in association with a pharmaceutically acceptable carrier.

5. A compound as claimed in any one of claims 1 to 3 for use in the treatment of CNS disorders in mammals.

6. The use of a compound as claimed in any one of claims 1 to 3 in the manufacture of a medicament for the treatment of CNS disorders in mammals.

7. A method for preparing a compound as claimed in any one of claims 1 to 3 comprising acylating an amine of formula (B)

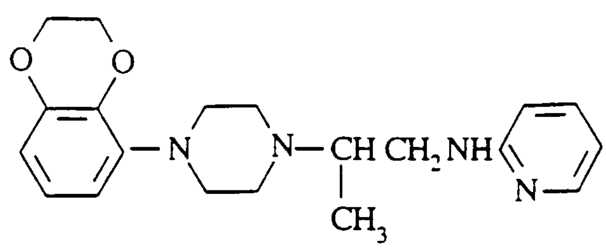

(B)

or a salt thereof,
with cyclohexanecarboxylic acid or an acylating derivative thereof.

8. A method as claimed in claim 7 wherein an acylating derivative is used in the form of an acid halide, azide, anhydride, imidazolide, activated ester or O-acyl urea obtained from a carbodiimide such as a dialkylcarbodiimide particularly cyclohexylcarbodiimide.

9. A compound of the formula (B):

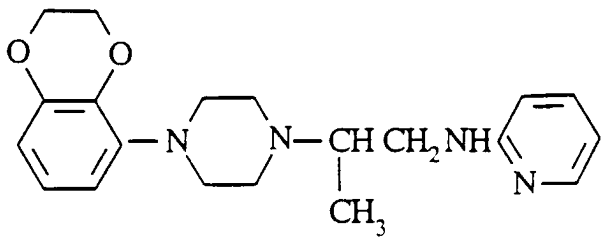

(B)

or a salt thereof.

**10.** A method for preparing a compound as claimed in claim 9 comprising the step of reducing a compound of formula (C):

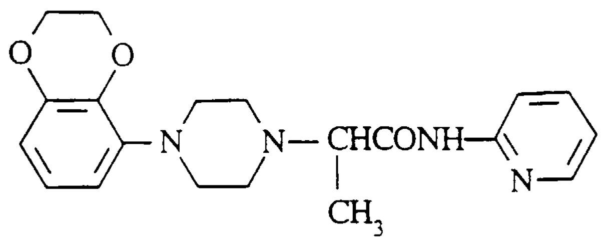

(C)

or a salt thereof.

**11.** A method for preparing a compound as claimed in claim 9 wherein an oxathiazolidine-2,2-dioxide of formula (D)

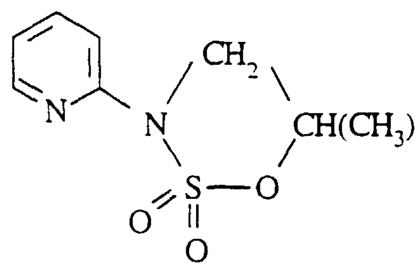

(D)

or a salt thereof,
is reacted with 1-(2,3-dihydro-1,4-benzodioxin-5-yl)piperazine.

**12.** A method for preparing a compound as claimed in any one of claims 1 to 3 which comprises alkylating an amide of formula (E)

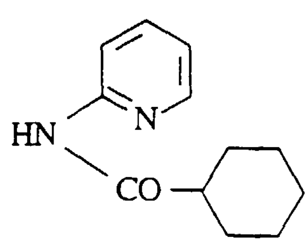

(E)

or a salt thereof,

with an alkylating agent providing the group (F)

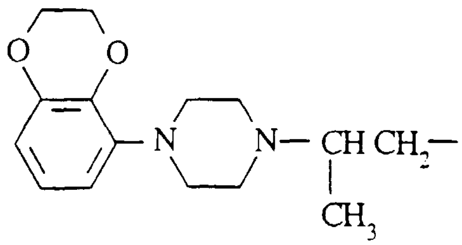

(F)

or a salt thereof.

**13.** A method for preparing a compound as claimed in any one of claims 1 to 3 comprising alkylating a compound of formula (G)

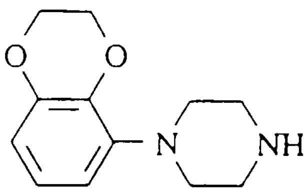

(G)

or a salt thereof,
with a compound of formula (H)

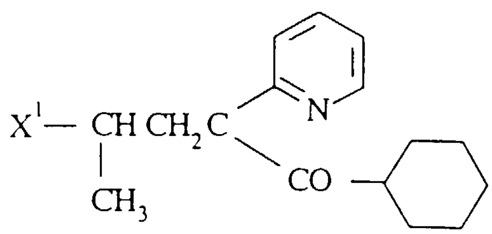

(H)

or a salt thereof,
(where $X^1$ is is a leaving group such as halogen or an alkyl- or aryl- sulphonyloxy group).

**Patentansprüche**

**1.** Verbindung der allgemeinen Formel (A):

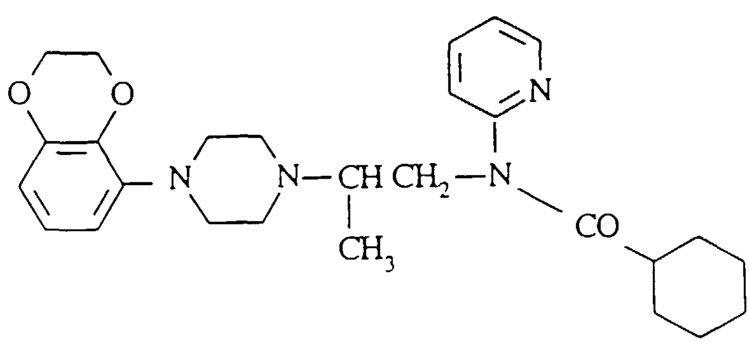

(A)

oder ein pharmazeutisch annehmbares Säureadditionssalz davon.

2. R-N-[1-[2-[1-[4-[5-(2,3-Dihydro[1,4]benzodioxinyl)]]-piperazinyl]]propyl]-N-(2-pyridyl)-cyclohexancarboxamid oder ein pharmazeutisch annehmbares Säureadditionssalz davon.

3. Verbindung wie in Anspruch 1 oder Anspruch 2 beansprucht, worin das Säureadditionssalz ein Salz, abgeleitet von Schwefel-, Salz-, Bromwasserstoff-, Phosphor-, Wein-, Fumar-, Malein-, Zitronen-, Essig-, Ameisen-, Methansulfon-, p-Toluolsulfon-, Oxal- oder Bernsteinsäure ist.

4. Pharmazeutische Zusammensetzung, umfassend eine Verbindung wie in einem der Ansprüche 1 bis 3 beansprucht, in Verbindung mit einem pharmazeutisch annehmbaren Träger.

5. Verbindung wie in einem der Ansprüche 1 bis 3 beansprucht, zur Verwendung bei der Behandlung von CNS-Störungen in Säugern.

6. Verwendung einer Verbindung wie in einem der Ansprüche 1 bis 3 beansprucht, in der Herstellung einer Arznei zur Behandlung von CNS-Störungen in Säugern.

7. Verfahren zum Herstellen einer Verbindung wie in einem der Ansprüche 1 bis 3 beansprucht, umfassend Acylieren eines Amins der Formel (B)

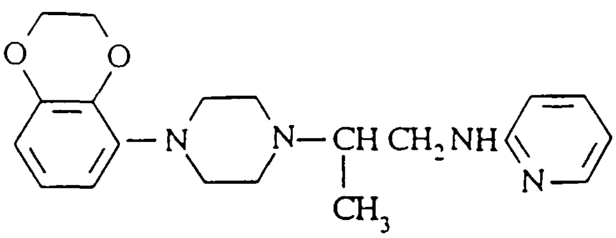

(B)

oder eines Salzes davon, mit Cyclohexancarbonsäure oder einem acylierenden Derivat davon.

8. Verfahren wie in Anspruch 7 beansprucht, worin ein acylierendes Derivat in Form eines Säurehalogenids, Azids, Anhydrids, Imidazolids, aktivierten Esters oder O-Acylharnstoffs, erhalten aus einem Carbodiimid, wie einem Dialkylcarbodiimid, insbesondere Cyclohexylcarbodiimid verwendet wird.

9. Verbindung der Formel (B)

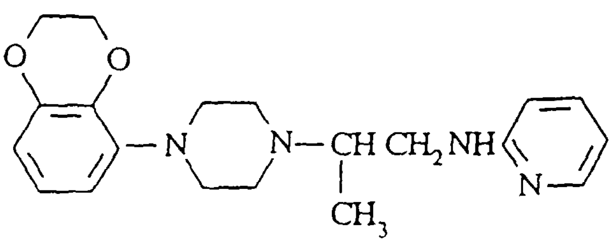

(B)

oder ein Salz davon.

10. Verfahren zum Herstellen einer Verbindung wie in Anspruch 9 beansprucht, umfassend den Schritt: Reduzieren einer Verbindung der Formel (C):

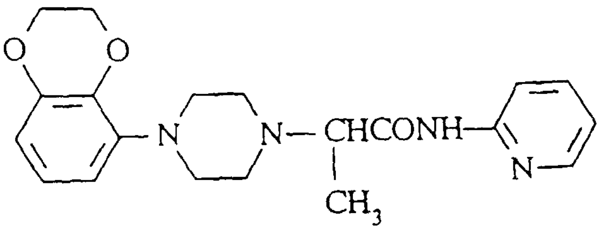

(C)

oder eines Salzes davon.

**11.** Verfahren zum Herstellen einer Verbindung wie in Anspruch 9 beansprucht, worin ein Oxathiazolidin-2,2-dioxid der Formel (D)

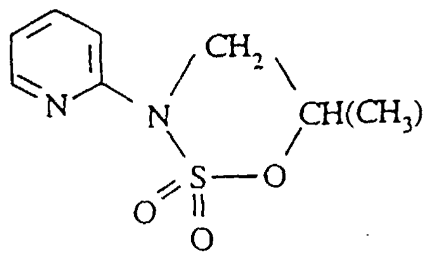

(D)

oder ein Salz davon mit 1-(2,3-Dihydro-1,4-benzodioxin-5-yl)piperazin umgesetzt wird.

**12.** Verfahren zum Herstellen einer Verbindung wie in einem der Ansprüche 1 bis 3 beansprucht, welches umfasst: Alkylieren eines Amids der Formel (E)

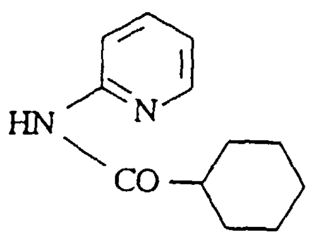

(E)

oder eines Salzes davon, mit eines Alkylierungsmittel, vorsehend die GruDoe (F)

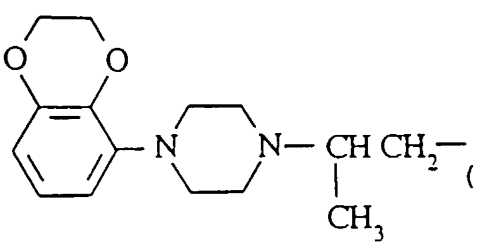

(F)

oder ein Salz davon.

**13.** Verfahren zum Herstellen einer Verbindung wie in einem der Ansprüche 1 bis 3 beansprucht, umfassend Alkylieren einer Verbindung der Formel (G)

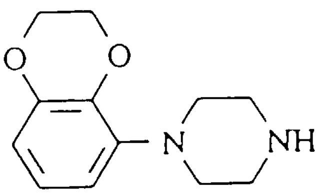

(G)

oder ein Salz davon, mit einer Verbindung der Formel (H)

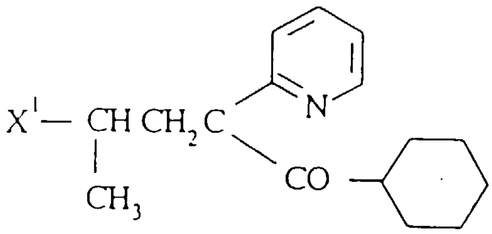

(H)

oder einem Salz davon (worin $X^1$ eine austretende Gruppe wie Halogen oder eine Alkyl- oder Aryl-Sulfonyloxy-Gruppe darstellt).

## Revendications

**1.** Composé de la formule générale (A) :

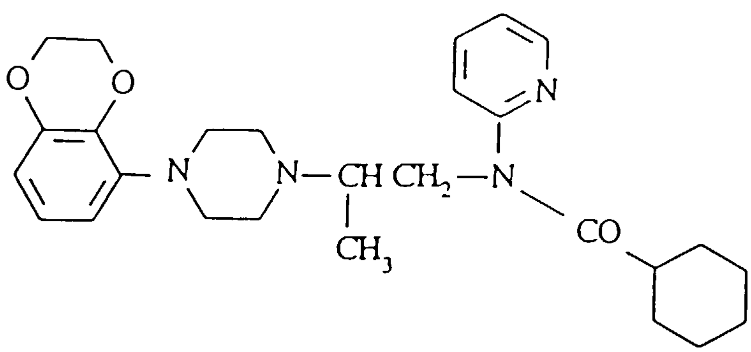

(A)

ou un de ses sels d'addition avec des acides pharmaceutiquement acceptables.

**2.** R-N-[1-[2-[1-[4-[5-(2,3-dihydro[1,4] benzodioxinyl)]pipérazinyl]propyl]-N-(2-pyridyl)-cyclohexanecarboxamide ou un de ses sels d'addition avec des acides pharmaceutiquement acceptables.

**3.** Composé selon la revendication 1 ou 2, dans lequel le sel d'addition avec un acide est un sel tiré des acides sulfurique, chlorhydrique, bromhydrique, phosphorique, tartrique, fumarique, maléique, citrique, acétique, formique, méthanesulfonique, p-toluènesulfonique, oxalique ou succinique.

**4.** Composition pharmaceutique comprenant un composé selon l'une quelconque des revendications 1 à 3 en association avec un véhicule pharmaceutiquement acceptable.

**5.** Composé selon l'une quelconque des revendications 1 à 3 utilisé dans le traitement des troubles du système nerveux central chez les mammifères.

**6.** Utilisation d'un composé selon l'une quelconque des revendications 1 à 3 dans la fabrication d'un médicament pour le traitement des troubles du système nerveux central chez les mammifères.

**7.** Procédé de préparation d'un composé selon l'une quelconque des revendications 1 à 3, comprenant l'acylation d'une amine de formule (B) :

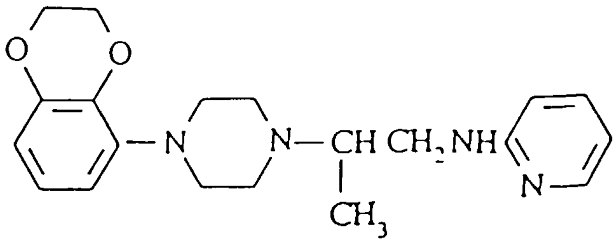

(B)

ou un de ses sels,
avec un acide cyclohexanecarboxylique ou un de ses dérivés d'acylation.

**8.** Procédé selon la revendication 7, dans lequel on utilise un dérivé d'acylation sous la forme d'un halogénure d'acide, d'un azide, d'un anhydride, d'un imidazolide, d'un ester activé ou d'urée de O-acyle obtenue à partir d'un carbodiimide tel qu'un dialkylcarbodiimide, en particulier le cyclohexylcarbodiimide.

**9.** Composé de la formule (B) :

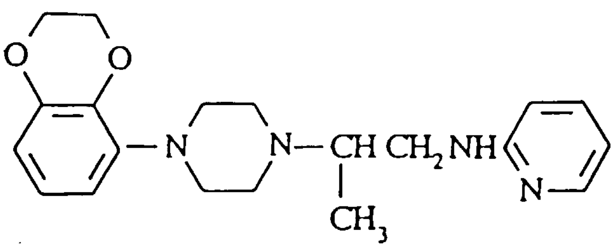

(B)

ou un de ses sels.

**10.** Procédé de préparation d'un composé selon la revendication 9, comprenant l'étape de réduction d'un composé de formule (C) :

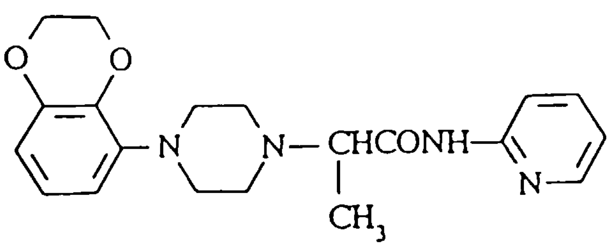

(C)

ou d'un de ses sels.

**11.** Procédé de préparation d'un composé selon la revendication 9, dans lequel on fait réagir un 2,2-dioxyde d'oxathiazolidine de formule (D) :

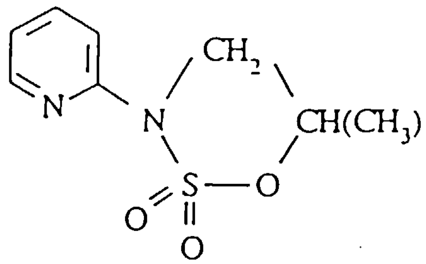

(D)

ou un de ses sels,
avec de la 1-(2,3-dihydro-1,4-benzodioxin-5-yl)pipérazine.

**12.** Procédé de préparation d'un composé selon l'une quelconque des revendications 1 à 3, comprenant l'alkylation d'un amide de formule (E) :

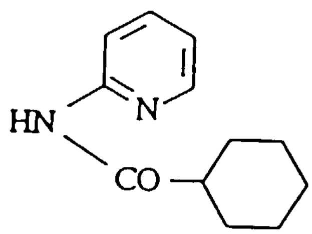

(E)

ou d'un de ses sels,
avec un agent d'alkylation qui donne le groupe (F) :

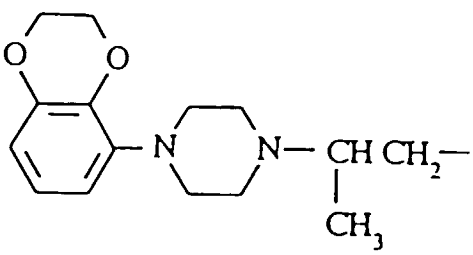

(F)

ou un de ses sels.

**13.** Procédé de préparation d'un composé selon l'une quelconque des revendications 1 à 3, comprenant l'alkylation d'un composé de formule (G) :

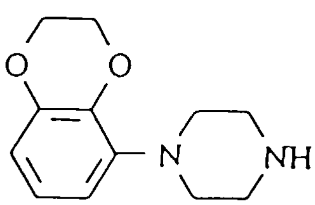

(G)

ou d'un de ses sels,

avec un composé de formule (H) :

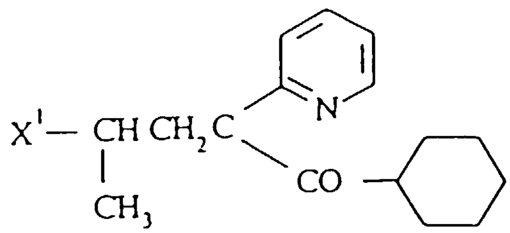

(H)

ou un de ses sels,

(où $X^1$ est un groupe partant tel qu'un halogène ou un groupe alkyl- ou arylsulfonyloxy.